(19) Europäisches Patentamt
European Patent Office
Office européen des brevets

(11) EP 4 674 849 A1

(12) **EUROPEAN PATENT APPLICATION**
published in accordance with Art. 153(4) EPC

(43) Date of publication:
**07.01.2026 Bulletin 2026/02**

(21) Application number: **24763878.6**

(22) Date of filing: **27.02.2024**

(51) International Patent Classification (IPC):
***C07D 417/14*** *(2006.01)* ***C09B 23/10*** *(2006.01)*
***G01N 21/64*** *(2006.01)*

(52) Cooperative Patent Classification (CPC):
**C07D 417/14; C09B 23/10; G01N 21/64**

(86) International application number:
**PCT/JP2024/006954**

(87) International publication number:
**WO 2024/181398 (06.09.2024 Gazette 2024/36)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **02.03.2023 JP 2023032301**

(71) Applicants:
- **Keio University**
  **Tokyo, 108-8345 (JP)**
- **HITACHI HIGH-TECH CORPORATION**
  **Tokyo 105-6409 (JP)**

(72) Inventors:
- **HANAOKA, Kenjiro**
  **Tokyo 105-8512 (JP)**
- **SASAKI, Eita**
  **Tokyo 105-8512 (JP)**
- **SUZUKI, Ami**
  **Tokyo 105-8512 (JP)**
- **AKIMOTO, Kaoru**
  **Tokyo 105-6409 (JP)**

(74) Representative: **Müller-Boré & Partner Patentanwälte PartG mbB**
**Friedenheimer Brücke 21**
**80639 München (DE)**

(54) **FLUORESCENT DYE FOR DETECTING NUCLEIC ACID**

(57) Provided is a compound of the following formula (I):

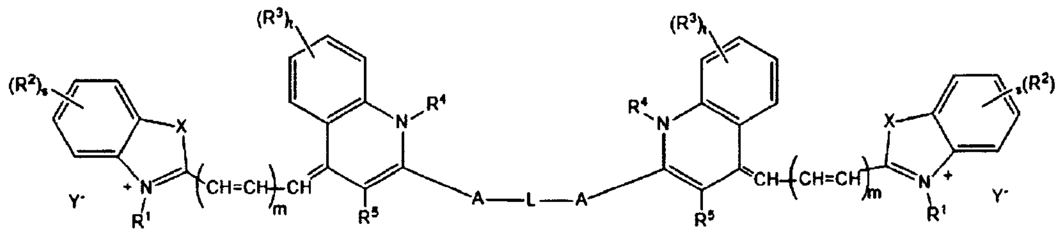

(I)

wherein $R^1$, $R^2$, $R^3$, $R^4$, $R^5$, X, m, s, t, A, L, and $Y^-$ are as defined in the specification.



Processed by Luminess, 75001 PARIS (FR)

## Description

### Technical Field

The present invention relates to a fluorescent dye for detecting nucleic acids and a method for detecting nucleic acids using the fluorescent dye.

### Background Art

Detection of nucleic acids using fluorescent dyes has been well known, and cyanine dyes are often used as fluorescent dyes. For example, PTL 1 discloses a novel asymmetric cyanine dye derivative and a method for nucleic acid detection by binding the derivative to nucleic acids. NPL 1 discloses the performance of SYBR (trademark) Green I, which is a typical commercially available asymmetric cyanine dye, as a DNA detection reagent. NPL 2 discloses the performance of SYBR (trademark) Gold, which is a typical commercially available asymmetric cyanine dye, as a DNA detection reagent.

### Citation List

#### Patent Literature

PTL 1: U.S. Patent No. 5,658,751

#### Non-patent Literature

NPL 1: J. Fluoresc. (2012) 22, 1189-1199
NPL 2: Nucleic Acid Research (2021) 49, 5143-5158

### Summary of Invention

#### Technical Problem

An object of the present invention is to provide a fluorescent dye capable of detecting nucleic acids with high sensitivity, and a method for detecting nucleic acids using the fluorescent dye.

#### Solution to Problem

The present invention encompasses embodiments described below.

Item 1.

A compound of the following formula (I):

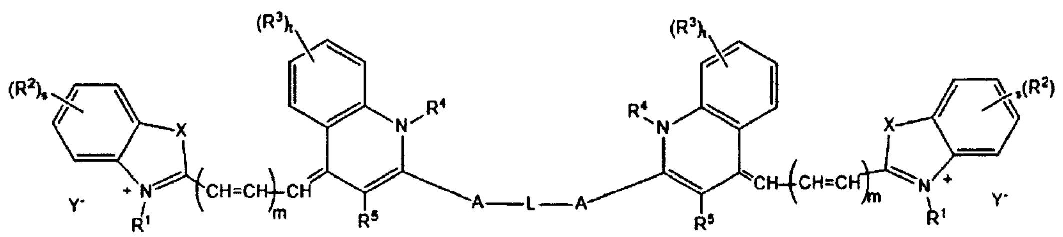

(I)

wherein

- each $R^1$ is independently $C_1$-$C_6$ alkyl;
- each $R^2$ is independently a group selected from the group consisting of $C_1$-$C_6$ alkyl and halogen;
- each X is S or O;
- each $R^3$ is independently a group selected from the group consisting of $C_1$-$C_6$ alkyl and halogen;

each $R^4$ is a phenyl group;
each $R^5$ is independently a group selected from the group consisting of hydrogen, $C_1$-$C_6$ alkyl, and halogen;
each m is 0, 1, or 2;
each s is independently 0, 1, 2, 3, or 4;
each t is independently 0, 1, 2, 3, or 4;
each A is any one of the following:

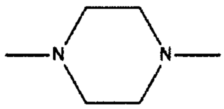
,
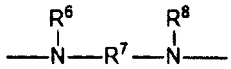

wherein $R^6$ and $R^8$ are independently hydrogen or $C_1$-$C_6$ alkyl, and $R^7$ is $-(CH_2)_r-$ wherein r is an integer ranging from 2 to 6;
L is $-CO-(CH_2-O-CH_2)_n-CO-$ wherein n is an integer ranging from 2 to 6, $-CO-(CH_2CH_2-O-CH_2CH_2)_p-CO-$ wherein p is an integer ranging from 2 to 6, or $-(CH_2)_q-$ wherein q is an integer of 2 to 6; and
each $Y^-$ is a counter anion.

Item 2.

The compound according to Item 1, which is a compound of the following formula (Ia):

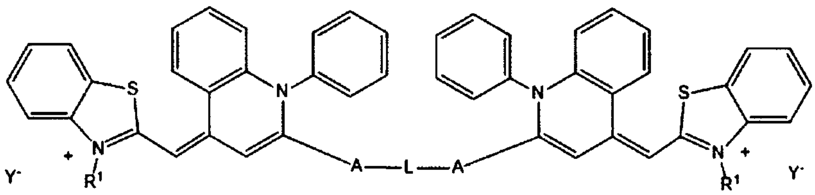

(Ia)

wherein

each $R^1$ is independently methyl or ethyl;
each A is any one of the following:

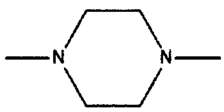
,
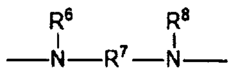

wherein $R^6$ and $R^8$ are independently hydrogen or $C_1$-$C_6$ alkyl, and $R^7$ is $-(CH_2)_r-$ wherein r is an integer of 2 to 6;
L is $-CO-(CH_2-O-CH_2)_n-CO-$ wherein n is an integer of 2 to 6), $-CO-(CH_2CH_2-O-CH_2CH_2)_p-CO-$ wherein p is an integer of 2 to 6, or $-(CH_2)_q-$ wherein q is an integer of 2 to 6; and
each $Y^-$ is a counter anion.

Item 3.

A fluorescent dye comprising the compound according to Item 1 or 2.

Item 4.

A compound of the following formula (II):

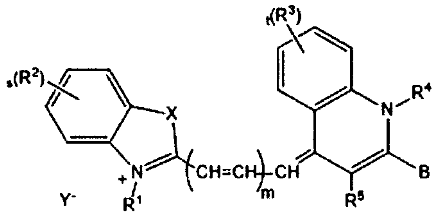

(II)

wherein

$R^1$ is $C_1$-$C_6$ alkyl;
$R^2$ is a group selected from the group consisting of $C_1$-$C_6$ alkyl and halogen;
X is S or O;
$R^3$ is a group selected from the group consisting of $C_1$-$C_6$ alkyl and halogen;
$R^4$ is a phenyl group;
$R^5$ is a group selected from the group consisting of hydrogen, $C_1$-$C_6$ alkyl, and halogen;
m is 0, 1, or 2;
s is 0, 1, 2, 3, or 4;
t is 0, 1, 2, 3, or 4;
B is any one of the following:

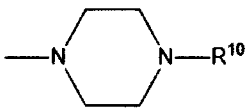
,
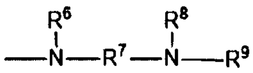

wherein $R^{10}$ is hydrogen or an ester represented by -COO-$R^{11}$ wherein $R^{11}$ is $C_1$-$C_6$ alkyl, $R^6$ and $R^8$ are independently hydrogen or $C_1$-$C_6$ alkyl, $R^7$ is -($CH_2$)- wherein r is an integer of 2 to 6, and $R^9$ is hydrogen or an ester represented by -COO-$R^{12}$ wherein $R^{12}$ is $C_1$-$C_6$ alkyl; and
$Y^-$ is a counter anion.

Item 5.

A kit for detecting a nucleic acid in a sample, comprising the compound according to Item 1 or 2.

Item 6.

A method for detecting a nucleic acid, comprising:

contacting a nucleic acid in a sample with the compound according to Item 1 or 2; and
detecting the fluorescence intensity of a complex of the nucleic acid and the compound.

Item 7.

The detection method according to Item 6, further comprising immobilizing the nucleic acid on a support, wherein detecting includes detecting the fluorescence intensity of a complex of the compound and the nucleic acid on the support.

Item 8.

The detection method according to Item 7,

wherein the contacting step comprises mixing the sample with the compound to prepare a liquid mixture, and
the immobilization step comprises immobilizing the liquid mixture on a gel through electrophoresis.

Item 9.

The method according to Item 6, wherein the nucleic acid is DNA or RNA.

## Advantageous Effects of Invention

The present invention provides a fluorescent dye capable of detecting nucleic acids with high sensitivity.

## Brief Description of Drawings

Fig. 1: Sensitivity of commercially available fluorescent dyes. (A): Structural formula of SYBR (registered trademark) Gold (left), and gel after electrophoresis with DNA stained with SYBR (registered trademark) Gold (right); (B): Structural formula of SYBR (registered trademark) Green I (left), and gel after electrophoresis with DNA stained with SYBR (registered trademark) Green I (right); and (C): Structural formula of GelGreen (trademark) (left), and gel after electrophoresis of DNA stained with GelGreen (trademark) (right). The arrows indicate the location of 140 pg of 50-bp DNA.

Fig. 2: Molecular design of fluorescent probes.

Fig. 3: (A) to (E): Absorption spectra of various dyes. (F) to (J): Fluorescence spectra of various dyes (excitation: 495 nm). (A) and (F): SYBR Gold; (B) and (G): SYBR Green I; (C) and (H): Compound 11; (D) and (I): Compound 13; and (E) and (J): Compound 14. The solid line represents after DNA addition, and the dashed line represents before DNA addition.

Fig. 4: DNA binding assay of various dyes. (A): SYBR Gold, (B): SYBR Green I, (C): Compound 11, (D): Compound 13, and (E): Compound 14.

Fig. 5: DNA electrophoresis test (post-staining). In order from left to right, gels of DNA stained with SYBR Gold, SYBR Green I, Compound 11, Compound 13, and Compound 14, respectively.

Fig. 6: DNA electrophoresis test (DNA pre-staining). In the top row, in order from left to right, gels of DNA stained with SYBR Gold, SYBR Green I, and Compound 11, respectively. In the bottom row, in order from left to right, gels of DNA stained with Compound 11 (twice the amount), Compound 13, and Compound 14, respectively.

Fig. 7: Electrophoresis results. The rectangular frames indicate the location of cfDNA (about 160 bp).

Lane 1: SYBR Gold (2 μM) + Ladder*, Lane 2: SYBR Gold (2 μM) + cfDNA, Lane 3: SYBR Gold (1 μM) + cfDNA, Lane 4: SYBR Gold (0.5 μM) + cfDNA, Lane 5: Compound 13 (2 μM) + Ladder*, Lane 6: Compound 13 (2 μM) + cfDNA, Lane 7: Compound 13 (1 μM) + cfDNA, Lane 8: Compound 13 (0.5 μM) + cfDNA, Lane 9: Compound 14 (2 μM) + Ladder*, Lane 10: Compound 14 (2 μM) + cfDNA, Lane 11: Compound 14 (1 μM) + cfDNA, and Lane 12: Compound 14 (0.5 μM) + cfDNA. The asterisks indicate Gene Ruler 50-bp DNA Ladder (Thermo Fisher Scientific) with DNA sizes of 1000, 900, 800, 700, 600, 500, 400, 300, 250, 200, 150, 100, and 50 bp.

Fig. 8: Intensity analysis of the electrophoresis results of Fig. 7 using ImageJ. (A): SYBR Gold, (B): Compound 13, and (C) Compound 14. Lanes 1 to 12 are the same as those in Fig. 7.

## Description of Embodiments

In the present specification, the terms "comprise" and "contain" also include the concepts of consisting essentially of and consisting of.

Before the completion of the present invention, the present inventors first studied the sensitivity of conventional fluorescent reagents. There are two types of methods for detecting DNA using gel electrophoresis: (1) a method in which DNA is run through a gel by electrophoresis and then the gel is stained with a fluorescent reagent (gel post-staining), and (2) a method in which DNA is stained with a fluorescent dye and then the stained DNA is run through a gel by electrophoresis (DNA pre-staining). Method (1) has high sensitivity. Method (2) has lower sensitivity than method (1) but is simple and rapid. Therefore, method (2) was performed by using Gene Ruler 50-bp DNA Ladder (Thermo Fisher Scientific) as a DNA sample; staining the DNA sample with SYBR (registered trademark) Gold (Invitrogen), SYBR (registered trademark) Green I (Invitrogen), and GelGreen (trademark) (Biotium) (Figs. 1 (A) to (C), compounds shown on the left side), which are well-known nucleic acid fluorescent dyes (SYBR (registered trademark) Gold, SYBR (registered trademark) Green I, and GelGreen (trademark) may be referred to below as "SYBR Gold," "SYBR Green I," and "GelGreen," respectively, for simplicity and ease of reading); and performing electrophoresis to achieve the DNA amount shown in Table I to detect fluorescence (excitation wavelength: 455 to 485 nm, detection wavelength, exposure time: 1 second).

As a result, as shown in the photographs on the right side of Figs. 1 (A) to (C), the shorter the DNA length, the lower the fluorescence intensity. Further, as indicated by the arrows, almost no fluorescence corresponding to 140 pg of 50-bp DNA was observed in the lane in which the amount of DNA was smallest. These results demonstrated that existing fluorescent dyes have insufficient sensitivity to detect short DNA, such as ctDNA, in the DNA pre-staining method.

Table I: Amount of DNA used in electrophoresis (pg/lane)

| bp | 1/10 | 1/50 | 1/250 |
|---|---|---|---|
| 150 | 7500 | 1500 | 300 |
| 50, 100, 150, 200 | 3500 | 700 | 140 |

The inventors thus thought that, as shown in Fig. 2, in the case of conventional fluorescent dyes, since fluorescent dye 2 interacts with DNA 1 at only one site (diagram on the left), maintaining the state in which fluorescent dye 2 is stably inserted into DNA 1 becomes difficult when DNA 1 has a shorter length, and then thought that if linker 3 of an appropriate length that binds two fluorescent dyes 2 is introduced to cause a single molecule of fluorescent dye 2 to interact with DNA 1 at two sites, a stable complex would be formed, which would increase the binding ability of fluorescent dye 2 to DNA 1 and improve the sensitivity of detection for DNA 1. The present invention has thus been accomplished.

According to one aspect of the present invention, a compound of the following formula (I) is provided.

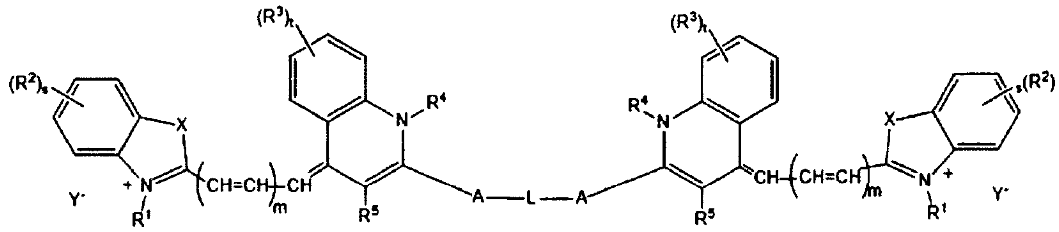

(I)

In the formula,

each $R^1$ is independently $C_1$-$C_6$ alkyl;
each $R^2$ is independently a group selected from the group consisting of $C_1$-$C_6$ alkyl and halogen;
each X is S or O;
each $R^3$ is independently a group selected from the group consisting of $C_1$-$C_6$ alkyl and halogen;
each $R^4$ is a phenyl group;
each $R^5$ is independently a group selected from the group consisting of hydrogen, $C_1$-$C_6$ alkyl, and halogen;
each m is 0, 1, or 2;
each s is independently 0, 1, 2, 3, or 4;
each t is independently 0, 1, 2, 3, or 4;
each A is any one of the following:

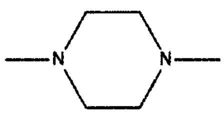

,

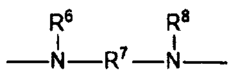

wherein $R^6$ and $R^8$ are independently hydrogen or $C_1$-$C_6$ alkyl, and $R^7$ is $-(CH_2)_r-$ wherein r is an integer ranging from 2 to 6;
L is $-CO-(CH_2-O-CH_2)_n-CO-$ wherein n is an integer of 2 to 6, $-CO-(CH_2CH_2-O-CH_2CH_2)_p-CO-$ wherein p is an integer of 2 to 6, or $-(CH_2)_q-$ wherein q is an integer of 2 to 6; and
each $Y^-$ is a counter anion.

The compound of formula (I) is a compound in which two monomers are linked via linker L and can bind to a nucleic acid at two sites. Therefore, this compound has improved ability to bind to a nucleic acid and can form a complex with a nucleic acid more stably, compared with a compound formed of a monomer that can bind to a nucleic acid molecule only at one site. The compound of formula (I) can be used as a fluorescent dye for nucleic acid detection.

Examples of $C_1$-$C_6$ alkyl for $R^1$ include, but are not limited to, methyl, ethyl, propyl, isopropyl, butyl, isobutyl, sec-butyl, tert-butyl, pentyl, isopentyl, neopentyl, tert-pentyl, hexyl, and isohexyl.

In a preferred embodiment, each $R^1$ is methyl or ethyl.

Examples of $C_1$-$C_6$ alkyl for each $R^2$ include, but are not limited to, methyl, ethyl, propyl, isopropyl, butyl, isobutyl, sec-butyl, tert-butyl, pentyl, isopentyl, neopentyl, tert-pentyl, hexyl, and isohexyl. Examples of halogen for each $R^2$ include fluorine, chlorine, bromine, and iodine.

In one embodiment, s is 0. In another embodiment, each s is independently 1, 2, 3, or 4, and each $R^2$ is

independently a group selected from the group consisting of methyl and halogen. Examples of halogen include chlorine, bromine, and iodine.

In one embodiment, X is O (oxygen). In another embodiment, X is S.

Examples of $C_1$-$C_6$ alkyl for each $R^3$ include, but are not limited to, methyl, ethyl, propyl, isopropyl, butyl, isobutyl, sec-butyl, tert-butyl, pentyl, isopentyl, neopentyl, tert-pentyl, hexyl, and isohexyl. Examples of halogen for each $R^3$ include fluorine, chlorine, bromine, and iodine.

In one embodiment, t is 0. In another embodiment, each t is independently 1, 2, 3, or 4, and each $R^3$ is independently a group selected from the group consisting of methyl and halogen. Examples of halogen include chlorine, bromine, and iodine.

Each $R^4$ is a phenyl group that may be unsubstituted or substituted. When substituted, the phenyl group may be substituted with $C_1$-$C_6$ alkyl, amino, or halogen, without limitation. Examples of $C_1$-$C_6$ alkyl include, but are not limited to, methyl, ethyl, propyl, isopropyl, butyl, isobutyl, sec-butyl, tert-butyl, pentyl, isopentyl, neopentyl, tert-pentyl, hexyl, and isohexyl.

Examples of $C_1$-$C_6$ alkyl for each $R^5$ include, but are not limited to, methyl, ethyl, propyl, isopropyl, butyl, isobutyl, sec-butyl, tert-butyl, pentyl, isopentyl, neopentyl, tert-pentyl, hexyl, and isohexyl.

In one embodiment, each $R^5$ is hydrogen.

In one embodiment, m is 0.

In one embodiment, A is

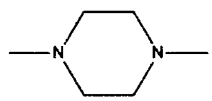

In another embodiment, A is

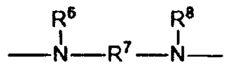

wherein $R^6$ and $R^8$ are independently hydrogen or $C_1$-$C_6$ alkyl, and $R^7$ is $-(CH_2)_r-$ wherein r is an integer of 2 to 6. $R^6$ and $R^8$ are preferably independently hydrogen, methyl, or ethyl.

In one embodiment, L is $-CO-(CH_2-O-CH_2)_n-CO-$ wherein n is an integer of 2 to 6. In another embodiment, L is $-(CH_2)_q-$wherein q is an integer of 2 to 6.

In one embodiment, examples of the counter anion for $Y^-$include, but are not limited to, halogen ions (e.g., $Cl^-$, $Br^-$, and $I^-$), alkyl sulfate ions (e.g., methyl sulfate ion and ethyl sulfate ion), organic sulfonate ions (e.g., methanesulfonate, p-toluenesulfonate, $CF_3SO_3^-$, and $(CF_3SO_2)_2N^-$), perchlorate ion ($ClO_4^-$), and $TsO^-$. The halogen ion is preferably $SbF_6^-$, $AsF_6^-$, $PF_6^-$, $BF_4^-$, $(FSO_2)_2N^-$, $CF_3SO_2^-$, or $CF_3CO_2^-$, more preferably $BF_4^-$, $CF_3SO_2^-$, or $CF_3CO_2^-$, and further preferably $CF_3CO_2^-$.

In one embodiment, the compound of formula (I) is a compound of the following formula (Ia).

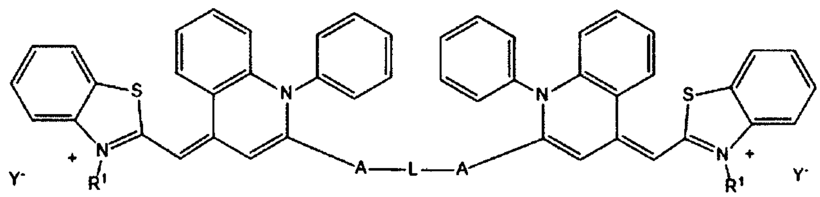

**(Ia)**

In the formula,

each $R^1$ is independently methyl or ethyl;
each A is any one of the following:

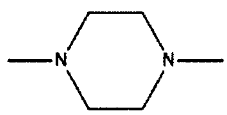

,

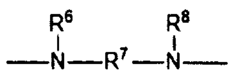

wherein $R^6$ and $R^8$ are independently hydrogen or $C_1$-$C_6$ alkyl, and $R^7$ is $-(CH_2)_r-$ wherein r is an integer of 2 to 6;
L is $-CO-(CH_2-O-CH_2)_n-CO-$ wherein n is an integer of 2 to 6, $-CO-(CH_2CH_2-O-CH_{2C}H_2)_p-CO-$ wherein p is an integer

of 2 to 6, or $-(CH_2)_q-$ wherein q is an integer of 2 to 6; and
each $Y^-$ is a counter anion.

In one embodiment, A is

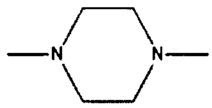

In another embodiment, A is

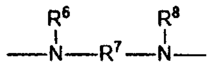

wherein $R^6$ and $R^8$ are independently hydrogen or $C_1$-$C_6$ alkyl, and $R^7$ is $-(CH_2)_r-$ wherein r is an integer of 2 to 6. $R^6$ and $R^8$ are preferably independently hydrogen, methyl, or ethyl.

In one embodiment, L is $-CO-(CH_2-O-CH_2)_n-CO-$ wherein n is an integer of 2 to 6. In another embodiment, L is $-(CH_2)_q-$wherein q is an integer of 2 to 6.

In one embodiment, examples of the counter anion for $Y^-$include, but are not limited to, halogen ions (e.g., $Cl^-$, $Br^-$, and $I^-$), alkyl sulfate ions (e.g., methyl sulfate ion and ethyl sulfate ion), organic sulfonate ions (e.g., methanesulfonate, p-toluenesulfonate, $CF_3SO_3^-$, and $(CF_3SO_2)_2N^-$), perchlorate ion ($ClO_4^-$), and $TsO^-$. The halogen ion is preferably $SbF_6^-$, $AsF_6^-$, $PF_6^-$, $BF_4^-$, $(FSO_2)_2N^-$, $CF_3SO_2^-$, or $CF_3CO_2^-$, more preferably $BF_4^-$, $CF_3SO_2^-$, or $CF_3CO_2^-$, and further preferably $CF_3CO_2^-$.

According to one aspect of the present invention, a compound of the following formula (II) is provided.

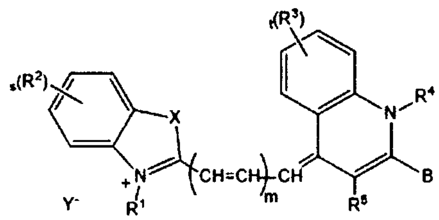

(II)

In the formula,

each $R^1$ is independently $C_1$-$C_6$ alkyl;
each $R^2$ is independently a group selected from the group consisting of $C_1$-$C_6$ alkyl and halogen;
X is S or O;
each $R^3$ is independently a group selected from the group consisting of $C_1$-$C_6$ alkyl and halogen;
each $R^4$ is a phenyl group;
each $R^5$ is independently a group selected from the group consisting of hydrogen, $C_1$-$C_6$ alkyl, and halogen;
m is 0, 1, or 2;
s is independently 0, 1, 2, 3, or 4;
t is independently 0, 1, 2, 3, or 4;
B is any one of the following:

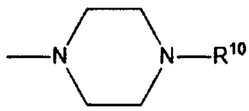

,

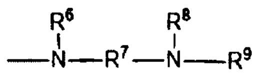

wherein $R^6$ and $R^8$ are independently hydrogen or $C_1$-$C_6$ alkyl, $R^7$ is $-(CH_2)_r-$ wherein r is an integer of 2 to 6, and $R^9$ is hydrogen or an ester represented by $-COO-R^{12}$ wherein $R^{12}$ is $C_1$-$C_6$ alkyl; and
$Y^-$ is a counter anion.

Examples of $C_1$-$C_6$ alkyl for $R^9$, $R^{11}$, and $R^{12}$ include, but are not limited to, methyl, ethyl, propyl, isopropyl, butyl, isobutyl, sec-butyl, tert-butyl, pentyl, isopentyl, neopentyl, tert-pentyl, hexyl, and isohexyl.

The compound of formula (II) is an intermediate used for the production of the compound of formula (I). In formula (II), $R^1$, $R^2$, $R^3$, $R^4$, $R^5$, $R^6$, $R^7$, $R^8$, X, $Y^-$, m, s, and t are as defined in formula (I).

In one embodiment, B is

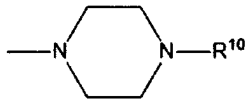

wherein $R^{10}$ is hydrogen or an ester represented by $-COO-R^{11}$ wherein $R^{11}$ is $C_1$-$C_6$ alkyl.

In another embodiment, B is

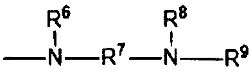

wherein $R^6$ and $R^8$ are independently hydrogen or $C_1$-$C_6$ alkyl, $R^7$ is $-(CH_2)_r-$ wherein r is an integer of 2 to 6, and $R^9$ is $C_1$-$C_6$ alkyl. $R^6$ and $R^8$ are preferably independently hydrogen, methyl, or ethyl.

In one embodiment, examples of the counter anion for $Y^-$include, but are not limited to, halogen ions (e.g., $Cl^-$, $Br^-$, and $I^-$), alkyl sulfate ions (e.g., methyl sulfate ion and ethyl sulfate ion), organic sulfonate ions (e.g., methanesulfonate, p-toluenesulfonate, $CF_3SO_3^-$, and $(CF_3SO_2)_2N^-$), perchlorate ion ($ClO_4^-$), and $TsO^-$. The halogen ion is preferably $SbF_6^-$, $AsF_6^-$, $PF_6^-$, $BF_4^-$, $(FSO_2)_2N^-$, $CF_3SO_2^-$, or $CF_3CO_2^-$, more preferably $BF_4^-$, $CF_3SO_2^-$, or $CF_3CO_2^-$, and further preferably $CF_3CO_2^-$.

In one embodiment, the compound of formula (II) is a compound of the following formula (IIa).

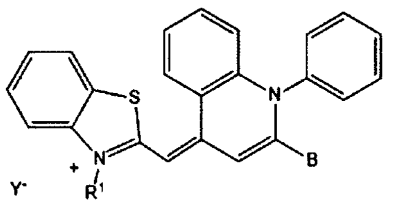

(IIa)

In the formula,

$R^1$ is methyl or ethyl;
B is any one of the following:

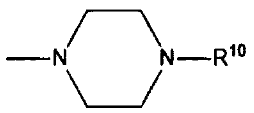 , 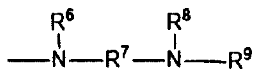

wherein $R^{10}$ is hydrogen or an ester represented by $-COO-R^{11}$ wherein $R^{11}$ is $C_1$-$C_6$ alkyl, $R^6$ and $R^8$ are independently hydrogen or $C_1$-$C_6$ alkyl, $R^7$ is $-(CH_2)_r-$ wherein r is an integer of 2 to 6, and $R^9$ is hydrogen or an ester represented by $-COO-R^{12}$ wherein $R^{12}$ is $C_1$-$C_6$ alkyl; and
$Y^-$ is a counter anion.

In one embodiment, examples of the counter anion $Y^-$include, but are not limited to, halogen ions (e.g., $Cl^-$, $Br^-$, and $I^-$), alkyl sulfate ions (e.g., methyl sulfate ion and ethyl sulfate ion), organic sulfonate ions (e.g., methanesulfonate, p-toluenesulfonate, $CF_3SO_3^-$, and $(CF_3SO_2)_2N^-$), perchlorate ion ($ClO_4^-$), and $TsO^-$. The halogen ion is preferably $SbF_6^-$, $AsF_6^-$, $PF_6^-$, $BF_4^-$, $(FSO_2)_2N^-$, $CF_3SO_2^-$, or $CF_3CO_2^-$, more preferably $BF_4^-$, $CF_3SO_2^-$, or $CF_3CO_2^-$, and further preferably $CF_3CO_2^-$.

When the compounds of formula (I) and formula (II) have isomers, such as optical isomers, stereoisomers, rotamers, and tautomers, all of such isomers and mixtures of isomers are included in the compounds of formula (I) and formula (II), unless otherwise specified. For example, when the compounds of formula (I) and formula (II) have optical isomers, optical isomers resolved from the racemate are also included in the compounds of formula (I) and formula (II), unless otherwise specified. These isomers can each be obtained as a single compound by known synthesis techniques and separation techniques (e.g., concentration, solvent extraction, column chromatography, and recrystallization).

The compounds of formula (I) and formula (II) may be individually crystalline, and single crystal forms and

polymorphic mixtures are also included in the compounds of the present invention or salts thereof. The crystals can be produced by crystallization according to known crystallization methods. The compounds of formula (I) and formula (II) may be solvates (e.g., hydrates) or non-solvates, both of which are included in the compounds of formula (I) and formula (II).

The following describes a method for producing the compound of formula (I).

First, the compound of formula (II), which is a monomer capable of binding to a nucleic acid, is produced based on the structure of an asymmetric cyanine dye, which is known to emit strong fluorescence upon interaction with DNA.

The compound of formula (II) can be synthesized, for example, according to Scheme 1 below. In Scheme 1, $R^1$, $R^2$, $R^4$, X, and $-(CH\text{-}CH)_m-$ are as defined in formula (II).

First, a compound represented by Compound (1), in which benzothiazole and quinoline are linked via a monoene or a polyene, can be synthesized by known methods, including, but not limited to, the method described in PTL 1, i.e., U.S. Patent No. 5,658,751.

First, Compound (1) is reacted with Compound (2), which is an alkyl ester of a secondary amine, in a solvent (e.g., 1,2-dichloroethane). The reaction temperature and time are not particularly limited. The reaction temperature is, for example, 30 to 70°C, and the reaction time is, for example, 30 minutes to 6 hours. In Compound (1), $R^1$, $R^2$, $R^4$, X, and $-(CH\text{-}CH)_m-$ are as defined in formula (II). In Compound (2), $R^{13}$ is

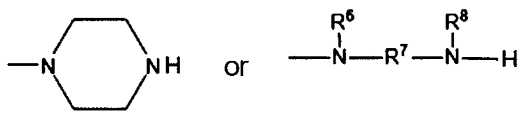

wherein $R^6$, $R^7$, and $R^8$ are as defined in formula (II). Next, the solvent is removed from the reaction solution, and the resulting product is purified to obtain Compound (3). In Compound (3), $R^{15}$ is

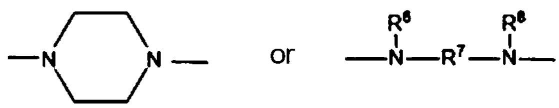

wherein $R^6$, $R^7$, and $R^8$ are as defined in formula (II).

Next, Compound (3) is hydrolyzed in an acid (e.g., trifluoroacetic acid) solution. The reaction temperature and time for hydrolysis are not particularly limited. The reaction temperature is, for example, -4°C to 4°C, and the reaction time is, for example, 5 minutes to 12 hours. Next, the solvent is removed from the reaction solution, and the resulting product is purified to obtain Compound (4).

In Scheme 1, distillation can be used to remove the solvent, and known methods, such as chromatography, can be used to purify the reaction product.

Compound (3) and Compound (4) are included in the compound of formula (II).

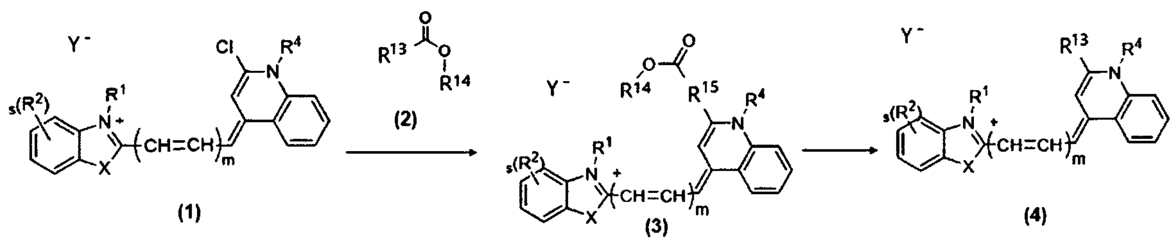

Scheme 1: Synthesis of Dye Backbone

Next, the monomers produced based on an asymmetric cyanine dye are linked with a linker to synthesize a compound of formula (I), which has two DNA binding sites in the molecule.

The compound of formula (I) can be synthesized, for example, according to Scheme 2 below. In Scheme 2, $R^1$, $R^2$, $R^4$, X, and $-(CH\text{-}CH)_m-$ are as defined in formula (I).

First, as a reference example, Compound (5), which is a monomer of a fluorescent dye, can be obtained from Compound (4). A compound represented by Compound (4) is dissolved in a solvent (e.g., dichloromethane), and triethylamine and acetyl chloride are added thereto, followed by stirring to induce the reaction. The reaction temperature and time are not particularly limited. The reaction temperature is, for example, -4°C to 4°C, and the reaction time is, for example, 5 to 60 minutes. Optionally, an additional solvent (e.g., methanol) may be further added and stirred. Next, the solvent is removed from the reaction solution, followed by purification to obtain Compound (5), which is a monomer of a

fluorescent dye.

Meanwhile, a compound represented by Compound (4) is dissolved in a solvent (e.g., N,N-dimethylformamide), and the components of a linker, i.e., alkanedioic acid (10) optionally having a polyether structure, 1-ethyl-3-(3-dimethylaminopropyl)carbodiimide (EDC), N-hydroxysuccinimide (HOSu), and N,N-diisopropylethylamine, are added thereto, followed by stirring. Next, the solvent is removed from the reaction solution, followed by purification to obtain a compound in which two molecules of Compound (4) are condensed via the linker. In Compound (10), f and g are each independently an integer of 1 to 3, j represents an integer of 2 to 6, and s and t are each 1 or 2.

For example, when alkanedioic acid (10) optionally having a polyether structure is 3,6-dioxaoctanedioic acid, Compound (6) is obtained. When alkanedioic acid (10) optionally having a polyether structure is 3,6,9-trioxaundecanedioic acid, Compound (7) is obtained. When alkanedioic acid (10) optionally having a polyether structure is 3,6,9,12-tetraoxatetradecanedioic acid, Compound (8) is obtained. In Compounds (6) to (8), $R^{16}$ is

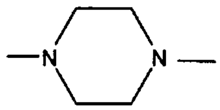

or

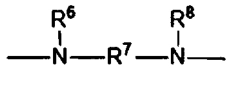

wherein $R^6$, $R^7$, and $R^8$ are as defined in formula (I).

In Scheme 2, distillation can be used to remove the solvent, and known methods, such as chromatography, can be used to purify the reaction product.

Compound (6), Compound (7), and Compound (8) are included in the compound of formula (I).

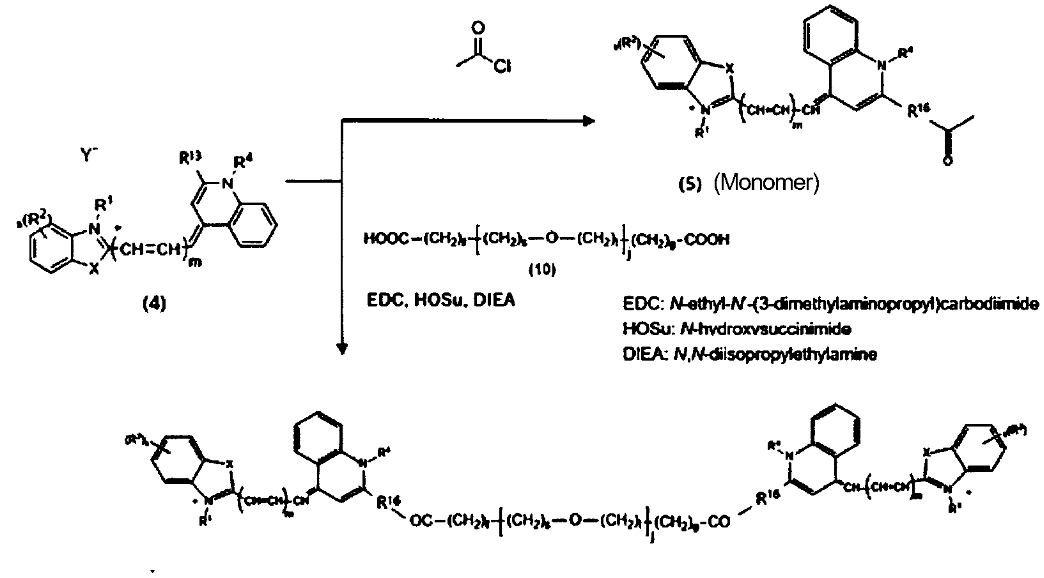


Scheme 2: Condensation of linker

(6) (n = 2)
(7) (n = 3)
(8) (n = 4)

Instead of the alkanedioic acid of Compound (10), it is also possible to use a compound having a substituent with high leaving ability (a leaving group), such as halogen, at both ends of an alkane to react with Compound (4) to form a linker; i.e., in formula (I), L is $-(CH_2)_q-$ wherein q is an integer of 2 to 6.

According to one aspect of the present invention, provided is a method for detecting a nucleic acid, comprising contacting a nucleic acid in a sample with the compound of formula (I), and detecting the fluorescence intensity of a complex of the nucleic acid and the compound of formula (I).

The sample is preferably a sample obtained from a living body, and preferably a sample obtained from a mammal. Mammals include, but are not limited to, humans, mice, rats, cows, horses, pigs, monkeys, dogs, cats, rabbits, goats, sheep, and the like, and are preferably humans, mice, or rats, and more preferably humans. The sample obtained from a living body may be a body fluid, tissue, or cell. Body fluids include blood (e.g., whole blood), blood cells, serum, plasma, pleural effusion, ascites fluid, cerebrospinal fluid, saliva, urine, stool, and the like. Tissues include the heart, arteries, kidneys, lungs, inner ear, paranasal sinuses, skin, nerves, blood vessels of other organs, and the like. Cells include cells obtained from such tissues. In terms of biological non-invasiveness, the sample obtained from a living body is preferably a body fluid, and more preferably blood, serum, or plasma. The nucleic acid may be DNA or RNA. The DNA may be single-stranded, double-stranded, triple-stranded, or quadruple-stranded DNA. The RNA may be single-stranded or double-stranded. The nucleic acid may be a natural polymer or synthetic polymer. According to this detection method, the use of the compound of formula (I) enables nucleic acid detection with high sensitivity.

The fluorescent dye comprising the compound of formula (I) is prepared for use by dissolving it in a solvent. The solvent may be, for example, water or a water-miscible organic solvent, such as dimethyl sulfoxide (DMSO), dimethyl formamide (DMF), or a lower alcohol (e.g., methanol or ethanol).

An effective amount of the compound of formula (I) is an amount sufficient to provide a fluorescent signal that enables nucleic acid detection. The concentration of the compound of formula (I) in the staining solution before mixing with a nucleic acid must be sufficient to give a fluorescent signal upon contact with a nucleic acid in a sample while minimizing the background fluorescence. The concentration of the fluorescent dye in the staining solution for application to a gel may be typically 0.1 μM to 10 μM, more typically 0.5 μM to 2 μM. The concentration of the fluorescent dye in the staining solution for detection and quantification of free nucleic acids may typically be 0.1 μM to 2 μM.

By mixing a nucleic acid-containing sample with the compound of formula (I) above, the nucleic acid comes into contact with the compound of formula (I) in the liquid mixture. The above cyanine dye compound of formula (I) has low intrinsic fluorescence when not associated with a nucleic acid, and becomes fluorescent upon binding to a nucleic acid; thus, the binding of the compound of formula (I) to the nucleic acid is detected as a detectable fluorescent signal.

The nucleic acid-containing sample and the compound of formula (I) may be mixed directly with each other, may be mixed in a separation solution, such as an electrophoresis solution, a sieving matrix, or a running buffer, or may be mixed in a precipitate (e.g., sucrose) or in a density gradient (e.g., a CsCl-containing density gradient). Alternatively, a nucleic acid immobilized on a matrix, such as a membrane, gel, or substrate, or on any other support may be contacted with the compound of formula (I). Examples of the membrane include a nitrocellulose membrane and a methylcellulose membrane. Examples of the gel include agarose gel, alginate hydrogel, and polyacrylamide gel. Examples of the substrate include a glass substrate or a quartz substrate.

The fluorescent dye may be contacted with the nucleic acid-containing sample before, during, or after nucleic acid separation by gel or capillary electrophoresis, gradient centrifugation, or other separation step, as necessary. For example, a nucleic acid in a sample may be separated by electrophoresis or gradient centrifugation, the fluorescent dye may then be allowed to bind to the separated nucleic acid, and the fluorescence intensity of a complex of the nucleic acid and the fluorescent dye (also referred to below as the "nucleic acid-dye complex") may be detected or measured to thereby detect the nucleic acid. Alternatively, a nucleic acid in a sample may be mixed with the fluorescent dye to allow binding of the nucleic acid to the fluorescent dye, the liquid mixture may then be separated by electrophoresis or gradient centrifugation, and the fluorescence intensity of a complex of the nucleic acid and the fluorescent dye may be detected or measured to thereby detect the nucleic acid.

When a support is used, a nucleic acid in a sample may be immobilized through electrophoresis, the fluorescent dye may then be allowed to bind to the immobilized nucleic acid, and the fluorescence intensity of the complex of the nucleic acid and the fluorescent dye may be detected or measured to thereby detect the nucleic acid (post-staining method). Alternatively, a nucleic acid in a sample may be mixed with the fluorescent dye to allow binding of the nucleic acid to the fluorescent dye, the liquid mixture may then be subjected to electrophoresis to separate the nucleic acid in the mixture onto a support by electrophoresis, and the fluorescence intensity of the complex of the nucleic acid and the fluorescent dye may be detected or measured to thereby detect the nucleic acid (pre-staining method).

Preferably, the fluorescent dye is mixed with a nucleic acid-containing sample or contacted with a nucleic acid at a temperature optimal for the biological activity of nucleic acids (usually between 5°C and 50°C) within the operating parameters of the fluorescent dye. For in vitro assays, the fluorescent dye is mixed with a nucleic acid-containing sample or contacted with a nucleic acid at room temperature (between 15 to 25°C). The time required for the staining of the nucleic acid with the fluorescent dye to reach equilibrium may vary depending on the conditions; in a preferred embodiment, the fluorescence of the nucleic acid-dye complex is observed within a few minutes, such as within one minute, of the nucleic acid coming into contact with the fluorescent dye.

The nucleic acid-dye complex formed by staining a nucleic acid in a sample with the fluorescent dye of the present invention comprises one or more fluorescent dye molecules and a non-covalently bound nucleic acid polymer. The nucleic acid-dye complex produces a fluorescent signal that is significantly enhanced, compared with the fluorescence of the fluorescent dye alone.

The fluorescence of the nucleic acid-dye complex is detected qualitatively or quantitatively by exciting the sample at a wavelength absorbed by the dye portion of the complex and measuring the emitted wavelength. The excitation wavelength is preferably within the range of 455 to 500 nm, and the detection wavelength is preferably within the range of 505 to 560 nm. Fluorescence is detected by visual inspection or by the use of existing equipment, such as CCD cameras, video cameras, photographic film, laser scanning devices, fluorometers, photodiodes, quantum counters, plate readers, epifluorescence microscopes, scanning microscopes, confocal microscopes, flow cytometers, and capillary electrophoresis detectors.

Once the dye-nucleic acid complex is formed, its presence can be detected as a fluorescent signal and used as an

indicator of the presence, location, or type of nucleic acid in the sample. In addition to use in such qualitative analyses, the nucleic acid in the sample can also be quantified by comparing the known relationship between the fluorescence of the nucleic acid-dye complex and the concentration of the nucleic acid in the sample.

The detection method of the present invention achieves high detection sensitivity and provides a strong fluorescent signal even when a small amount of nucleic acids is used. For example, it is possible to detect 1 ng or less, particularly about 100 pg to 800 pg, of double-stranded DNA for each band on an electrophoretic gel. By increasing the dye concentration, it is possible to detect an even smaller amount of nucleic acid; for example, it is possible to detect about several pg to several tens of pg of double-stranded DNA for each band on an electrophoretic gel.

In the detection method of the present invention, since a fluorescent dye having two DNA interaction sites in one molecule is used, it is possible to detect even short DNA with high sensitivity. For example, it is possible to detect cfDNA and DNA of 200 bp or less, in particular, tumor cDNA, i.e., double-stranded DNA of about 90 bp to 150 bp. Tumor-derived DNA fragments (ctDNA) circulating in the blood of a cancer patient reflect the DNA of the entire tumor, making it possible to understand the overall picture of the tumor. In addition, because their half-life is short, real-time tumor information can be obtained, which is useful for early cancer diagnosis and prognosis monitoring. However, ctDNA is short and the amount of ctDNA contained in blood samples is very small (Sci Transl Med., November 7, 2018; 10 (466)); thus, dyes for nucleic acid detection commonly used in conventional technology do not have sufficient sensitivity to achieve complete detection of ctDNA from various patients. The detection method using the fluorescent dye of the present invention can also be used for ctDNA analysis.

The method for nucleic acid detection according to the above aspect of the present invention can be used as a method for qualitatively or quantitatively analyzing target DNA or RNA containing a specific base sequence. Further, this method is useful for use in the fields of clinical diagnosis, such as disease diagnosis, disease diagnosis assistance, and genetic diagnosis.

According to one aspect of the present invention, provided is a kit for detecting a nucleic acid in a sample, comprising the compound of formula (I) described above.

The sample is preferably a sample obtained from a living body, and preferably a sample obtained from a mammal. Mammals include, but are not limited to, humans, mice, rats, cows, horses, pigs, monkeys, dogs, cats, rabbits, goats, sheep, and the like, and are preferably humans, mice, or rats, and more preferably humans. The sample obtained from a living body may be a body fluid, tissue, or cell. Body fluids include blood (e.g., whole blood), blood cells, serum, plasma, pleural effusion, ascites fluid, cerebrospinal fluid, saliva, urine, stool, and the like. Tissues include the heart, arteries, kidneys, lungs, inner ear, paranasal sinuses, skin, nerves, blood vessels of other organs, and the like. Cells include cells obtained from such tissues. In terms of biological non-invasiveness, the sample obtained from a living body is preferably a body fluid, and more preferably blood, serum, or plasma. The nucleic acid may be DNA or RNA. The DNA may be single-stranded or double-stranded DNA. The kit comprises the compound of formula (I), enabling nucleic acid detection with high sensitivity.

The use of the kit to perform the method for nucleic acid detection of the present invention described above enables nucleic acid detection. Therefore, the kit may further comprise instructions describing the method for nucleic acid detection. The kit may optionally further comprise nucleic acid fragments used as size markers and additional detection reagents (e.g., a staining compound specific only to DNA).

The disclosures of all patent applications and documents cited in the present specification are hereby incorporated by reference in their entirety.

Examples below are for illustrative purposes only and are not intended to limit the technical scope of the present invention in any way. The reagents used are commercially available products or are obtained or prepared according to conventional techniques in the related fields or procedures of published documents, unless otherwise specified.

## Examples

### Example 1: Synthesis of Dye Backbone

Compounds that serve as the backbone of a dye were synthesized in accordance with Scheme 3 below. Compound 8 was synthesized according to the following procedure with reference to, for example, the method described in PTL 1, i.e., U.S. Patent No. 5,658,751, using Compound 1 as a starting material.

#### (1) Synthesis of Compound 3

Compound 1 (2.42 g, 15.2 mmol), Compound 2 (3.70 g, 30.4 mmol), copper(II) acetate (2.75 g, 15.2 mmol), pyridine (1.23 mL, 15.2 mmol), triethylamine (2.12 mL, 7.6 mmol), and molecular sieves 3A (1 g) were added to 50 mL of dichloromethane, and the mixture was stirred at room temperature overnight. The reaction solution was filtered, and the solvent in the filtrate was distilled off using a rotary evaporator.

An aqueous saturated ammonium chloride solution was added to the residue, and the mixture was extracted with dichloromethane. The organic layer was dried over sodium sulfate, the solution was then filtered, and the solvent in the filtrate was distilled off using a rotary evaporator. The residue was purified by chromatography on an amino silica gel (linear gradient of ethyl acetate:hexane = 10:90 to 30:70) to obtain Compound 3 (1.7 g, 7.2 mmol, yield: 48%) as a white solid.

(2) Synthesis of Compound 4

Compound 3 (820 mg, 3.5 mmol) was dissolved in 35 mL of dichloromethane (super-dehydrated), and phosphoryl chloride (1.1 mg, 7.0 mmol) and a catalytic amount of N,N-dimethylformamide were added thereto, followed by refluxing at 50°C for 6 hours. After the temperature of the mixture was returned to room temperature, the solvent in the reaction liquid was distilled off using a rotary evaporator, and the residue was washed with a solvent in a ratio of ethyl acetate to diethyl ether of 1:1 to obtain a crudely purified product as Compound 4.

(3) Synthesis of Compound 7

Compound 5 (3.6 g, 20 mmol) and Compound 6 (3.1 mL, 20 mmol) were mixed and heated at 130°C for 3 hours, and then the temperature was returned to room temperature to obtain pale-yellow Compound 7 (7.2 g, 20 mmol).

(4) Synthesis of Compound 8

Compound 7 (900 mg, 2.45 mmol) was added to Compound 4 (crudely purified product, up to 3.5 mmol) and stirred vigorously at room temperature, triethylamine (6 mL) was then added thereto, and the mixture was stirred for another 5 minutes. To this reaction liquid, 15 mL of concentrated hydrochloric acid was added, and the mixture was stirred at 0°C for 30 minutes. Water was added to the dark-purple reaction mixture and extracted with dichloromethane. The organic layer was dried over sodium sulfate, the solution was then filtered, and the solvent in the filtrate was distilled off using a rotary evaporator. The residue was crudely purified by reverse-phase chromatography on a $C_{18}$ column (linear gradient of acetonitrile (containing 0.1% trifluoroacetic acid): water (containing 0.1% trifluoroacetic acid) = 10:90 to 30:70) to obtain a crudely purified product as Compound 8.

(5) Synthesis of Compound 9

1-(Tert-butoxycarbonyl) piperazine (1.2 g, 54 mmol) and 1,2-dichloroethane (20 mL) were added to the crudely purified product of Compound 8 (0.52 g), and the mixture was stirred at 50°C for 2 hours. After cooling to room temperature, the solvent in the reaction solution was distilled off using a rotary evaporator. Furthermore, the residue was crudely purified by chromatography on an amino silica gel (linear gradient of methanol: ethyl acetate = 0:100 to 50:50) to obtain a crudely purified product as Compound 9.

(6) Synthesis of Compound 10

Trifluoroacetic acid (3 mL) was added to the crudely purified product of Compound 9, the mixture was stirred at 0°C for 20 minutes, and the solvent in the reaction solution was distilled off using a rotary evaporator. The residue was purified by reverse-phase chromatography on a $C_{18}$ column (linear gradient of acetonitrile (containing 0.1% trifluoroacetic acid): water (containing 0.1% trifluoroacetic acid) = 1:99 to 100:0) to obtain Compound 10 (193 mg, 0.34 mmol, yield: 9.8%) as a red solid (4 steps from Compound 3 used as a starting material).

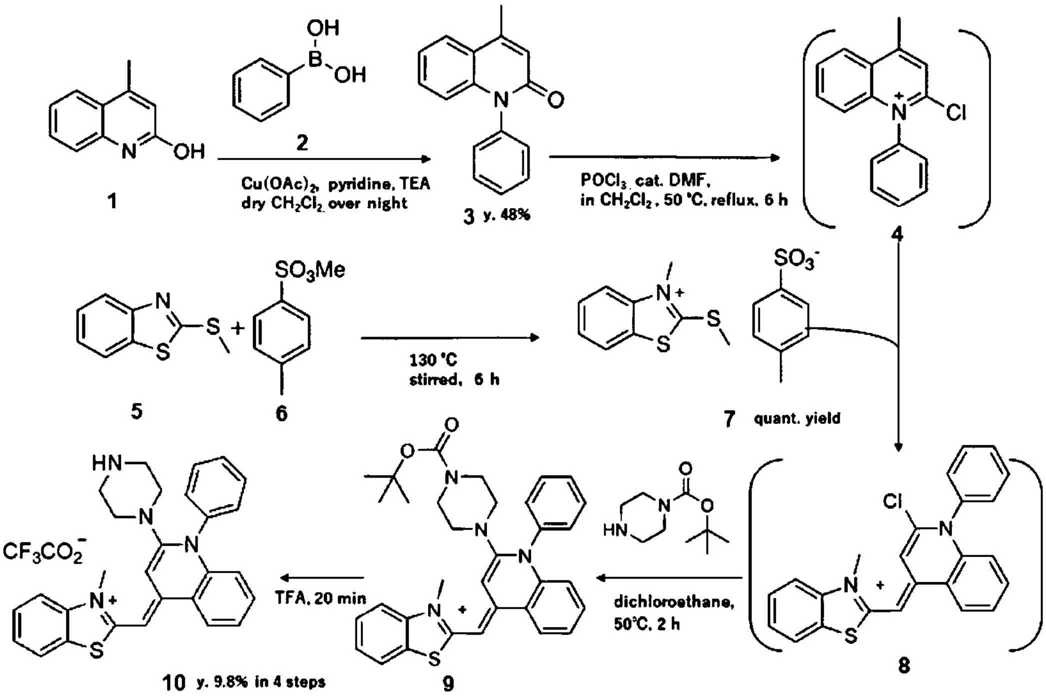


Scheme 3: Synthesis of dye backbone

Example 2: Condensation of Linker

The compounds synthesized in Example 1 were linked with a linker in accordance with Scheme 4, and novel fluorescent dyes having two DNA binding sites, which are included in the compound of formula (I) of the present invention, were successfully synthesized.

(1) Synthesis of Compound 11

Compound 10 (20 mg, 0.035 mmol) was dissolved in 3 mL of dichloromethane (super-dehydrated), and one drop of triethylamine and one drop of acetyl chloride were added thereto, followed by stirring at 0°C for 30 minutes. Then, 2 mL of methanol was added to the reaction liquid, and the mixture was stirred at room temperature for another 5 minutes. The solvent in the reaction solution was distilled off using a rotary evaporator, and the residue was purified by high-performance liquid chromatography on a $C_{18}$ column (linear gradient of acetonitrile (containing 0.1% trifluoroacetic acid): water (containing 0.1% trifluoroacetic acid) = 30:70 to 100:0) to obtain Compound 11 (16 mg, 0.026 mmol, yield: 75%) as a red solid.

(2) Synthesis of Compound 12

3,6-Dioxaoctanedioic acid (6.4 mg, 0.036 mmol), 1-ethyl-3-(3-dimethylaminopropyl) carbodiimide (46 mg, 0.24 mmol), N-hydroxysuccinimide (27.5 mg, 0.24 mmol), and N,N-diisopropylethylamine (80 μL, 0.46 mmol) were dissolved in 3 mL of N,N-dimethylformamide (super-dehydrated), and Compound 10 (54 mg, 0.096 mmol) dissolved in 3 mL of N,N-dimethylformamide (super-dehydrated) was added. The resulting mixture was then stirred at room temperature overnight. The solvent in the reaction solution was distilled off using a rotary evaporator, and the residue was purified by high-performance liquid chromatography on a $C_{18}$ column (linear gradient of acetonitrile (containing 0.1% trifluoroacetic acid): water (containing 0.10 trifluoroacetic acid) = 30:70 to 100:0) to obtain Compound 12 (7.1 mg, 0.056 mmol, yield: 16%) as a red solid.

(3) Synthesis of Compound 13

3,6,9-Trioxaundecanedioic acid (2.8 mg, 0.016 mmol), 1-ethyl-3-(3-dimethylaminopropyl)carbodiimide (4.0 mg, 0.021 mmol), N-hydroxysuccinimide (2.0 mg, 0.017 mmol), and N,N-diisopropylethylamine (8.0 μL, 0.046 mmol) were dissolved in 3 mL of N,N-dimethylformamide (super-dehydrated), and Compound 10 (17 mg, 0.030 mmol) dissolved in 3

mL of N,N-dimethylformamide (super-dehydrated) was added. The resulting mixture was then stirred at room temperature overnight. The solvent in the reaction solution was distilled off using a rotary evaporator, and the residue was purified by high-performance liquid chromatography on a $C_{18}$ column (linear gradient of acetonitrile (containing 0.1% trifluoroacetic acid): water (containing 0.1% trifluoroacetic acid) = 30:70 to 100:0) to obtain Compound 13 (3.3 mg, 0.0025 mmol, yield: 17%) as a red solid.

(4) Synthesis of Compound 14

3,6,9,12-Tetraoxatetradecanedioic acid (3.2 mg, 0.012 mmol), 1-ethyl-3-(3-dimethylaminopropyl) carbodiimide (7.4 mg, 0.039 mmol), and N-hydroxysuccinic acid were dissolved in 3 mL of N,N-dimethylformamide (super-dehydrated), and Compound 10 (17 mg, 0.030 mmol) dissolved in 3 mL of N,N-dimethylformamide (super-dehydrated) was added. The resulting mixture was stirred at room temperature overnight. The solvent in the reaction solution was distilled off using a rotary evaporator, and the residue was purified by high-performance liquid chromatography on a $C_{18}$ column (linear gradient of acetonitrile (containing 0.1% trifluoroacetic acid): water (containing 0.1% trifluoroacetic acid) = 30:70 to 100:0) to obtain Compound 14 (2.2 mg, 0.0016 mmol, yield: 14%) as a red solid.

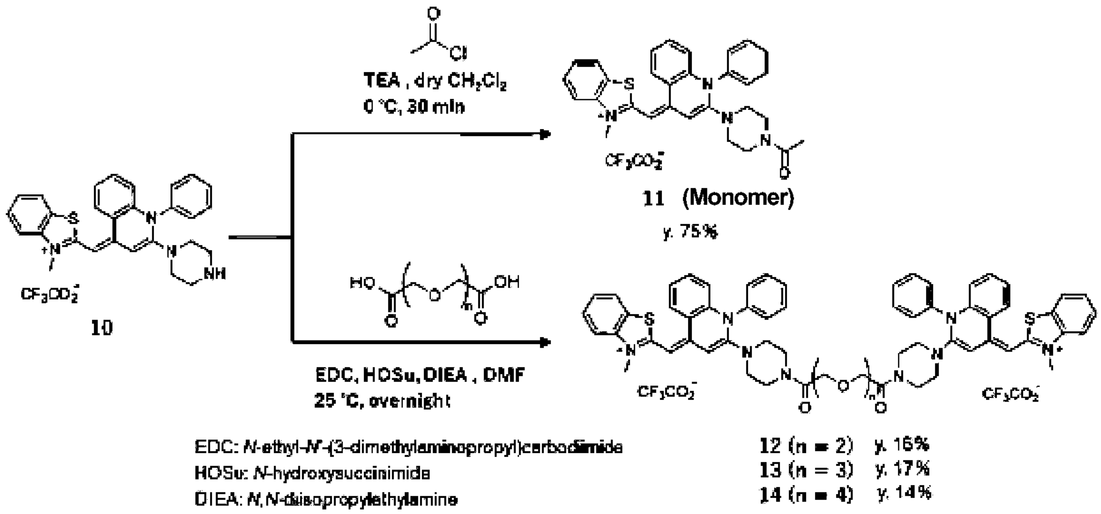


Scheme 4: Condensation of Linker

Example 3: Measurement of Absorption and Fluorescence Spectra of Various Fluorescent Dyes

SYBR Gold and SYBR Green I, as well as Compound 11, Compound 13, and Compound 14 produced in Example 2, were individually dissolved in 800 μL of TE buffer (pH: 8.0) to a concentration of about 1 μM, and the absorption and fluorescence spectra were measured using a quartz cell with an optical path length of 0.5 cm. Next, to each of the solutions, 10 μl of a solution of deoxyribonucleic acid (derived from salmon sperm) with a length of 500 to 1000 bp in TE buffer (970 ng/μl) was added, and the absorption and fluorescence spectra were measured in the same manner.

Results

As shown in Figs. 3 (H), (I), and (J), Compound 11, Compound 13, and Compound 14 showed a strong increase in fluorescence upon addition of DNA. Compound 11, Compound 13, and Compound 14 showed a 500-fold or greater increase in fluorescence at a wavelength of 520 nm upon binding to DNA. No significant difference in fluorescence intensity was observed due to the chain length of the linker.

Example 4: DNA Binding Assay of Various Fluorescent Dyes

Solutions of individual dyes of SYBR Gold, SYBR Green I, Compound 11, Compound 13, and Compound 14 (final concentrations: 200, 400, and 800 nM) and deoxyribonucleic acid (derived from salmon sperm) with a length of 500 to 1000 bp (final concentrations: 0.5, 1, 2, 3, 4, 6, 8.5, and 12 μM) in TE buffer (pH: 8.0) were prepared in 96-well plates, and the fluorescence intensity was measured with a plate reader (excitation wavelength: 475 nm, emission wavelength: 520 nm). The obtained results were fitted to the McGhee-von Hippel equation (Equation 1) to determine the dissociation constant

($K_d$) and the number of base pairs at the dye-binding site (N).

Equation 1

$$\frac{\alpha \cdot I}{c_{DNA}} = \frac{(c_{total} - \alpha \cdot I)}{K_d} \cdot \frac{1 - N \cdot \frac{\alpha \cdot I}{c_{DNA}}}{\left(1 - N \cdot \frac{\alpha \cdot I}{c_{DNA}} + \frac{\alpha \cdot I}{c_{DNA}}\right)}$$

**$K_d$: Dissociation constant**
**N: Number of base pairs to which dye binds**
**$\alpha$: Coefficient ($I$=$c_{bound}/\alpha$)**
**$I$: Fluorescence intensity**
**$c_{DNA}$: [DNA](M • bp)**
**$c_{total}$: [Dye](M)**

Results

Figs. 4 (A) to (E) are graphs showing DNA concentrations and the fluorescence intensity of each compound. Table 1 shows the dissociation constant $K_d$ and the number of base pairs at the dye-binding site N.

Compound 13 and Compound 14 interact with DNA at two DNA binding sites in the molecule, and therefore have improved ability to bind to DNA, compared with Compound 11, which interacts with DNA at only one site. As shown in Table 1, Compound 13 and Compound 14, which are dimers, had lower dissociation constant $K_d$ values, achieving an improvement in binding ability.

Compound 13 (n=3) showed the highest DNA binding ability. Compound 13 had a lower dissociation constant, compared to the commercially available fluorescent reagents. In addition, Compound 13 and Compound 14 showed an increase in the number of base pairs at the dye-binding site N, indicating the occurrence of intercalation into base pairs at two sites simultaneously.

**Table 1: Dissociation constant** $K_d$ of **various fluorescent dyes and number of base pairs at the dye-binding site N**

| | Dissociation constant $K_d$($\mu$M) | Binding site size N |
|---|---|---|
| SYBR Gold | 0.71±0.10 | 4.8±0.4 |
| SYBR Green I | 1.00±0.09 | 7.0±0.4 |
| 11 | 1.73±0.06 | 3.6±1.7 |
| 13 (n = 3) | 0.40±0.01 | 10.7±1.1 |
| 14 (n = 4) | 1.08±0.12 | 8.9±0.6 |

Example 5: DNA Electrophoresis Test - Post-staining Test

A solution containing Gene Ruler 50-bp DNA Ladder (Thermo Fisher Scientific) with 3500, 700, 140, 70, and 23 pg of DNA with lengths of 50, 100, 150, and 200 bp, respectively, was loaded onto an 8% polyacrylamide gel and subjected to electrophoresis at 100 V for 30 minutes in TBE buffer. After electrophoresis, the gel was stained with a solution of SYBR Gold, SYBR Green I, Compound 11, Compound 13, or Compound 14 in TE buffer (each at 1 $\mu$M) for 30 minutes, and subjected to fluorescence detection with a gel imager: iBright FL1500 Imaging System (Thermo Fisher Scientific) (excitation wavelength: 455 to 485 nm, emission wavelength: 508 to 557 nm, exposure time: 2 seconds).

Results

As shown in Fig. 5, the use of different dyes showed no significant difference in detection sensitivity. This is

presumably because the amount of dye relative to DNA was excessive under the above conditions. SYBR Gold showed a high level of background noise; however, Compound 11, Compound 13, and Compound 14 showed a lower level of background noise than SYBR Gold, showing a clearer difference from the fluorescence intensity at the time of DNA binding.

Example 6: DNA Electrophoresis Test - DNA Pre-staining Test

A solution containing Gene Ruler 50-bp DNA Ladder (Thermo Fisher Scientific) with 3500, 700, and 140 pg of DNA with lengths of 50, 100, 150, and 200 bp, respectively, and containing SYBR Gold, SYBR Green I, Compound 11, Compound 13, or Compound 14 at a final concentration of 0.67 μM was loaded in an amount of 6 μL onto an 8% polyacrylamide gel, and subjected to electrophoresis at 100 V for 30 minutes in TBE buffer. After electrophoresis, the gel was subjected to fluorescence detection with the gel imager, i.e., the iBright FL1500 Imaging System (Thermo Fisher Scientific) (excitation wavelength: 455 to 485 nm, emission wavelength: 508 to 557 nm, exposure time: 0.4 seconds), and the fluorescence intensity of each band was quantitatively analyzed.

Results

As shown in Fig. 6 and Table 2, Compound 13 and Compound 14, which are dimers, showed improved DNA detection ability, demonstrating improved ability to bind to DNA, compared with Compound 11, which is a monomer. In addition, Compound 13 (n = 3) had greater detection sensitivity than Compound 14 (n = 4); thus, the chain length of Compound 13 may be optimal. Compound 13 had a lower dissociation constant compared to the commercially available fluorescent reagents and successfully showed strong fluorescence in electrophoresis tests under DNA pre-staining conditions.

**Table 2: Relative fluorescence intensity of bands**

| | SYBR Gold | SYBR Green I | 11 | 11 (×2) | 13 n = 3 | 14 n = 4 |
|---|---|---|---|---|---|---|
| 100 bp (700 pg) | 1.00 | 0.96 | 0.92 | 1.35 | 1.53 | 1.03 |
| 100 bp (140 pg) | 1.00 | 1.04 | 0.05 | 0.47 | 1.18 | 1.21 |
| 50 bp (700 pg) | 1.00 | 0.62 | 0.41 | 0.93 | 1.60 | 1.45 |
| 50 bp (140 pg) | 1.00 | 0.96 | 0.05 | 0.08 | 1.61 | 0.23 |

Example 7: DNA Electrophoresis Test - DNA Pre-staining Test

A solution containing Gene Ruler 50-bp DNA Ladder (Thermo Fisher Scientific) with 10 ng of DNA in total and containing SYBR Gold, Compound 13, or Compound 14 at a final concentration of 2 μM, or a solution containing a cfDNA sample extracted from an actual specimen of a cancer patient with 3.9 ng of DNA in total and containing SYBR Gold, Compound 13, or Compound 14 at a final concentration of 0.5 μM, 1 μM, or 2 μM was loaded in an amount of 4 μL onto a 12% polyacrylamide gel, and subjected to electrophoresis at 350 V for 30 minutes in TBE buffer. After electrophoresis, the gel was irradiated with a 470-nm LED light built into a gel photography device (Bio-Pyramid, Mecan Imaging Inc.), and photographed with a digital camera (XZ-2, Olympus Corporation) (exposure time: 8 seconds).

Results

As shown in the rectangular frames in Fig. 7, bands around 160 bp, characteristic of cfDNA/ctDNA, were stained. Quantitative analysis of the fluorescence intensities of the bands using ImageJ revealed that the fluorescence intensities of the peak around 160 bp for cfDNA were almost the same between Compound 13 and Compound 14, and the fluorescence intensities of both of these compounds were stronger than that of SYBR Gold (Fig. 8). The results indicated that Compound 13 (n=3) and Compound 14 (n=4), which have been developed this time, are capable of detecting cfDNA/ctDNA bands with higher sensitivity, compared with when the commercially available SYBR Gold was used as a detection reagent.

Accordingly, Compounds 13 and 14 were demonstrated to be capable of detecting cfDNA (or ctDNA) with higher sensitivity than SYBR Gold even in a test in which an actual specimen sample (cfDNA extracted from a cancer patient) was used.

## Claims

1. A compound of the following formula (I):

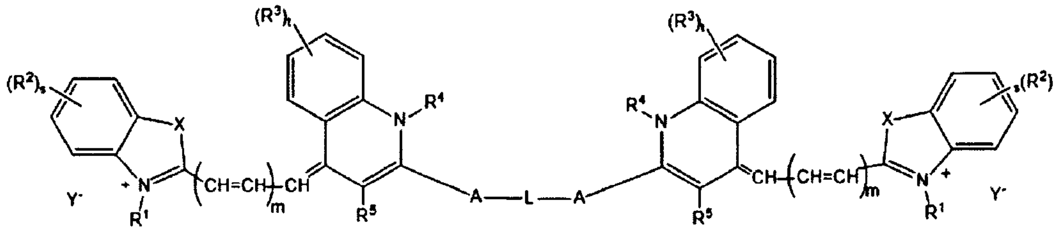

(I)

wherein

each $R^1$ is independently $C_1$-$C_6$ alkyl;
each $R^2$ is independently a group selected from the group consisting of $C_1$-$C_6$ alkyl and halogen;
each X is S or O;
each $R^3$ is independently a group selected from the group consisting of $C_1$-$C_6$ alkyl and halogen;
each $R^4$ is a phenyl group;
each $R^5$ is independently a group selected from the group consisting of hydrogen, $C_1$-$C_6$ alkyl, and halogen;
each m is 0, 1, or 2;
each s is independently 0, 1, 2, 3, or 4;
each t is independently 0, 1, 2, 3, or 4;
each A is any one of the following:

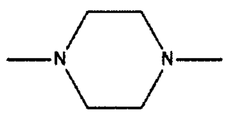

,

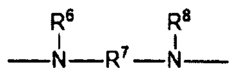

wherein $R^6$ and $R^8$ are independently hydrogen or $C_1$-$C_6$ alkyl, and $R^7$ is $-(CH_2)_r-$ wherein r is an integer ranging from 2 to 6;
L is $-CO-(CH_2-O-CH_2)_n-CO-$ wherein n is an integer ranging from 2 to 6, $-CO-(CH_2CH_2-O-CH_2CH_2)_p-CO-$ wherein p is an integer ranging from 2 to 6, or $-(CH_2)_q-$ wherein q is an integer of 2 to 6; and
each $Y^-$ is a counter anion.

2. The compound according to claim 1, which is a compound of the following formula (Ia):

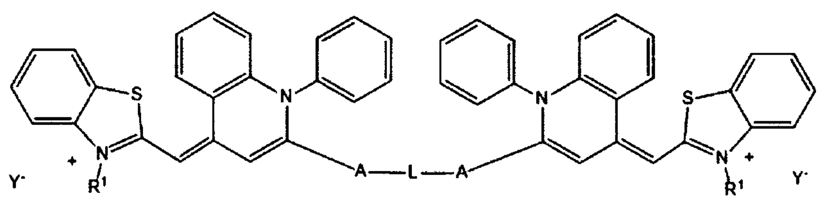

(Ia)

wherein

each $R^1$ is independently methyl or ethyl;
each A is any one of the following:

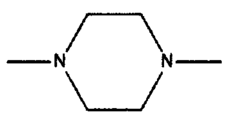

,

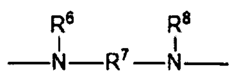

wherein $R^6$ and $R^8$ are independently hydrogen or $C_1$-$C_6$ alkyl, and $R^7$ is $-(CH_2)_r-$ wherein r is an integer of 2 to 6;
L is $-CO-(CH_2-O-CH_2)_n-CO-$ wherein n is an integer of 2 to 6), $-CO-(CH_2CH_2-O-CH_2CH_2)_p-CO-$ wherein p is an

integer of 2 to 6, or $-(CH_2)_q-$ wherein q is an integer of 2 to 6; and
each $Y^-$ is a counter anion.

**3.** A fluorescent dye comprising the compound according to claim 1 or 2.

**4.** A compound of the following formula (II):

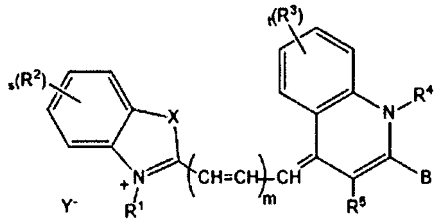

(II)

wherein

$R^1$ is $C_1$-$C_6$ alkyl;
$R^2$ is a group selected from the group consisting of $C_1$-$C_6$ alkyl and halogen;
X is S or O;
$R^3$ is a group selected from the group consisting of $C_1$-$C_6$ alkyl and halogen;
$R^4$ is a phenyl group;
$R^5$ is a group selected from the group consisting of hydrogen, $C_1$-$C_6$ alkyl, and halogen;
m is 0, 1, or 2;
s is 0, 1, 2, 3, or 4;
t is 0, 1, 2, 3, or 4;
B is any one of the following:

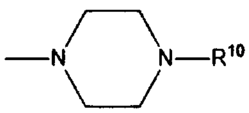

,

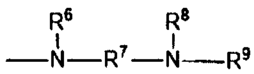

wherein $R^{10}$ is hydrogen or an ester represented by $-COO-R^{11}$ wherein $R^{11}$ is $C_1$-$C_6$ alkyl, $R^6$ and $R^8$ are independently hydrogen or $C_1$-$C_6$ alkyl, $R^7$ is $-(CH_2)_r-$ wherein r is an integer of 2 to 6, and $R^9$ is hydrogen or an ester represented by $-COO-R^{12}$ wherein $R^{12}$ is $C_1$-$C_6$ alkyl; and
$Y^-$ is a counter anion.

**5.** A kit for detecting a nucleic acid in a sample, comprising the compound according to claim 1 or 2.

**6.** A method for detecting a nucleic acid, comprising:

contacting a nucleic acid in a sample with the compound according to claim 1 or 2; and
detecting the fluorescence intensity of a complex of the nucleic acid and the compound.

**7.** The detection method according to claim 6, further comprising immobilizing the nucleic acid on a support, wherein detecting the fluorescence intensity includes detecting the fluorescence intensity of a complex of the compound and the nucleic acid on the support.

**8.** The detection method according to claim 7,

wherein the contacting step comprises mixing the sample with the compound to prepare a liquid mixture, and the immobilization step comprises immobilizing the liquid mixture on a gel through electrophoresis.

**9.** The method according to claim 6, wherein the nucleic acid is DNA or RNA.

Fig. 1

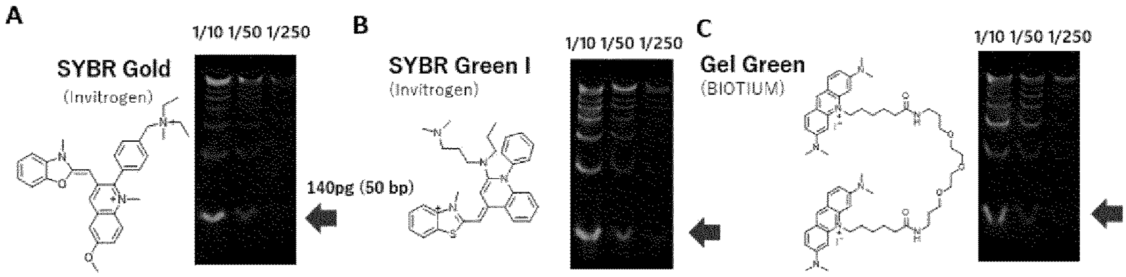


Fig. 2

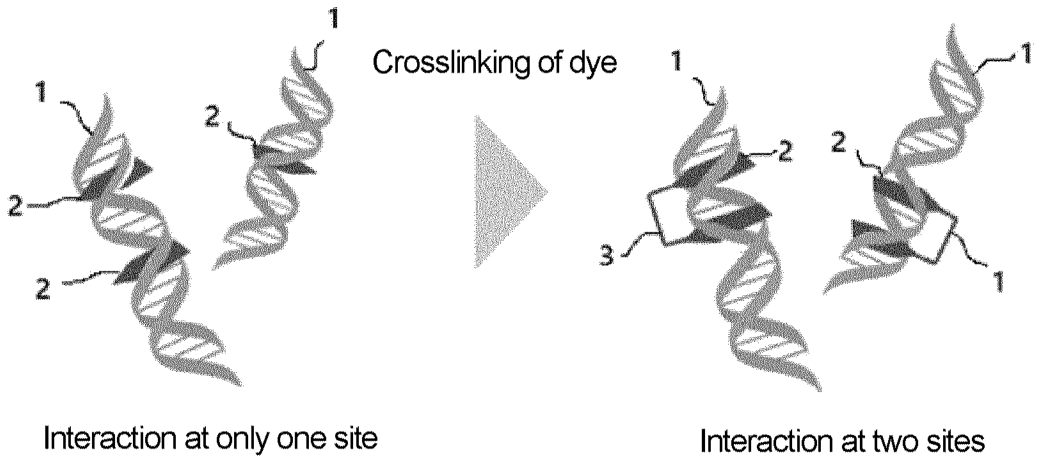


Increased
fluorescence

Fig. 3

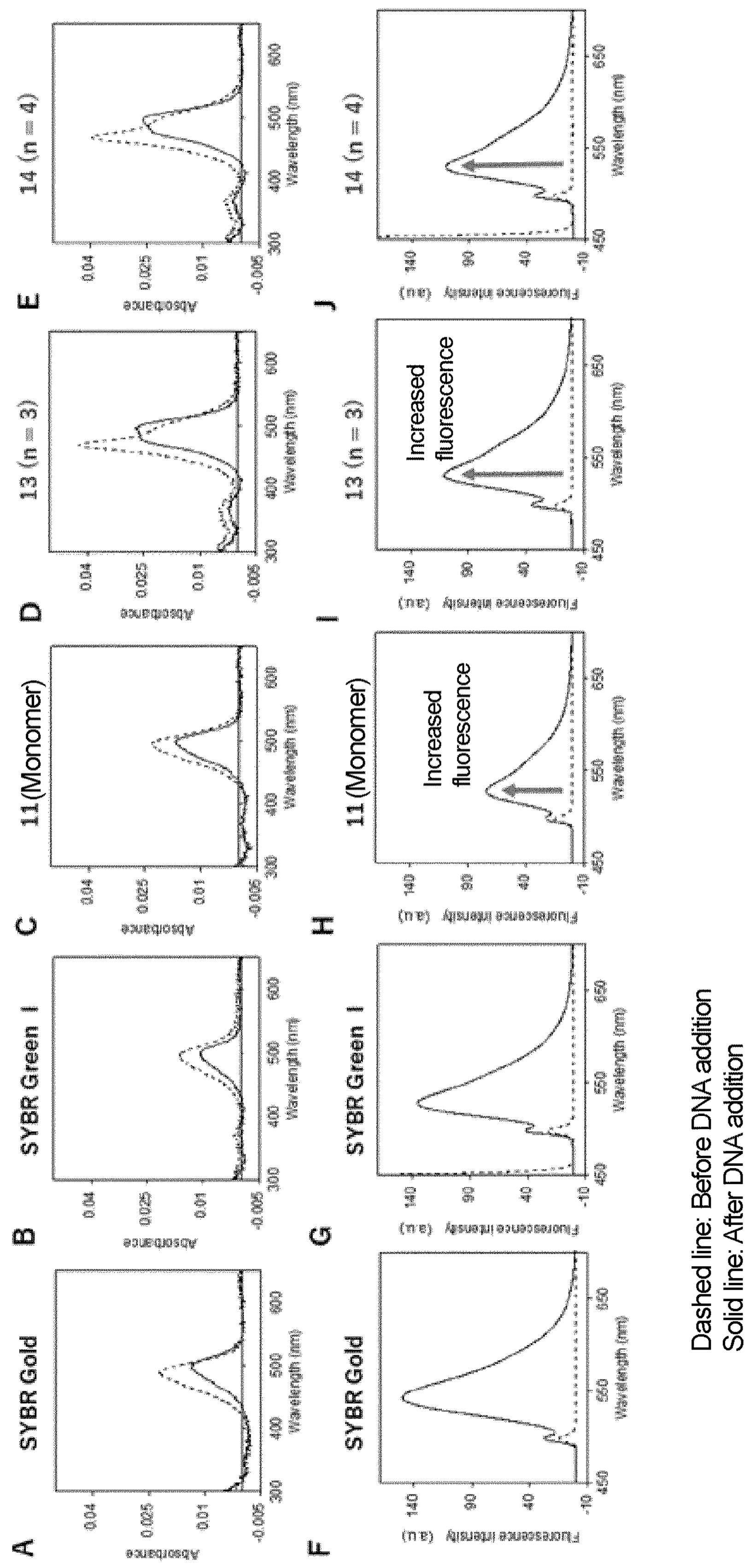
A
SYBR Gold
B
SYBR Green I
C
11(Monomer)
D
13 (n = 3)
E
14 (n = 4)
F
SYBR Gold
G
SYBR Green I
H
11(Monomer)
I
13 (n = 3)
J
14 (n = 4)
Absorbance
Wavelength (nm)
Fluorescence intensity (a.u)
Increased fluorescence
Increased fluorescence
Dashed line: Before DNA addition
Solid line: After DNA addition

Fig. 4

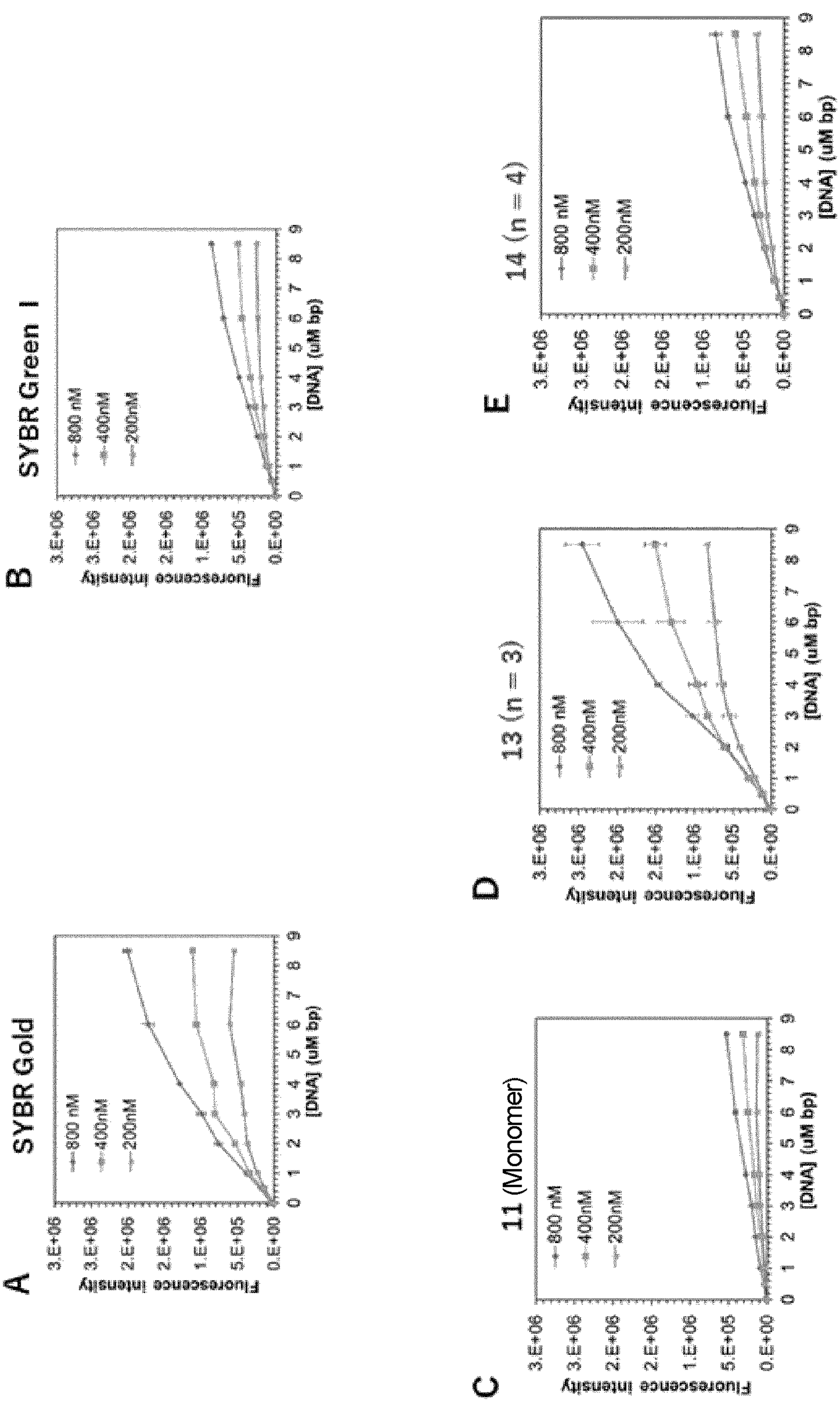
A
SYBR Gold
B
SYBR Green I
C
11 (Monomer)
D
13 (n = 3)
E
14 (n = 4)
800 nM
400nM
200nM
Fluorescence intensity
[DNA] (uM bp)
3.E+06
3.E+06
2.E+06
2.E+06
1.E+06
5.E+05
0.E+00
0 1 2 3 4 5 6 7 8 9

Fig. 5

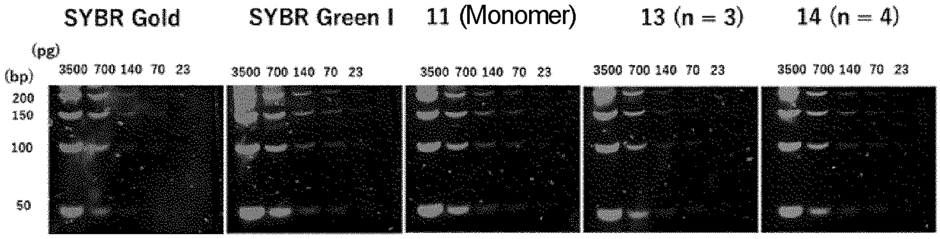


Fig. 6

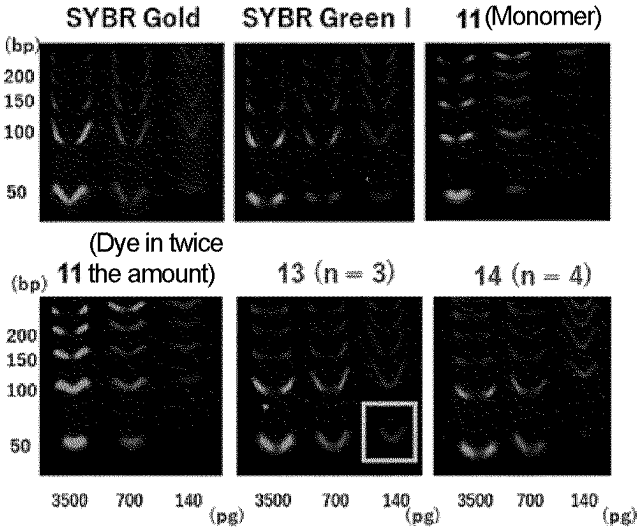


Fig. 7

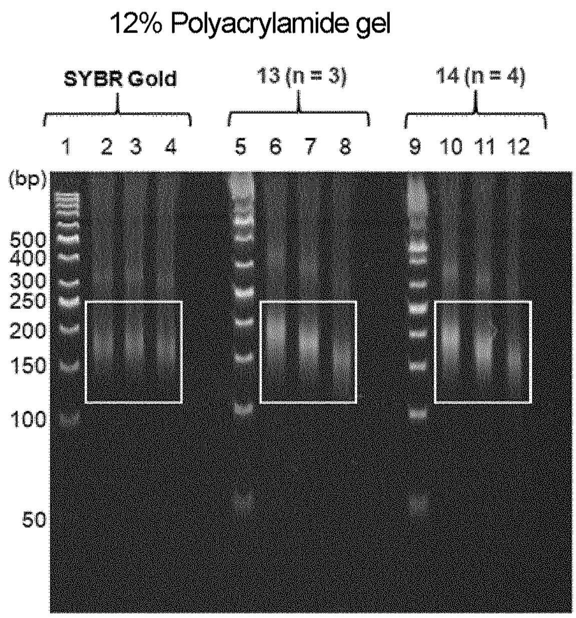

Fig. 8

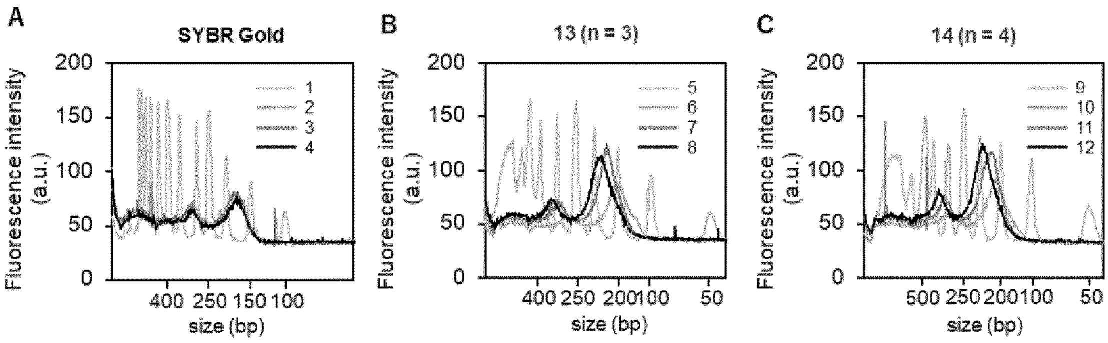
A
SYBR Gold
Fluorescence intensity (a.u.)
200
150
100
50
0
1
2
3
4
400 250 150 100
size (bp)
B
13 (n = 3)
Fluorescence intensity (a.u.)
5
6
7
8
400 250 200 100 50
size (bp)
C
14 (n = 4)
Fluorescence intensity (a.u.)
9
10
11
12
500 250 200 100 50
size (bp)

**INTERNATIONAL SEARCH REPORT**

International application No. **PCT/JP2024/006954**

**A. CLASSIFICATION OF SUBJECT MATTER**

***C07D 417/14***(2006.01)i; ***C09B 23/10***(2006.01)i; ***G01N 21/64***(2006.01)i
FI: C07D417/14; G01N21/64 F; C09B23/10 CSP

According to International Patent Classification (IPC) or to both national classification and IPC

**B. FIELDS SEARCHED**

Minimum documentation searched (classification system followed by classification symbols)

C07D417/14; C09B23/10; G01N21/64

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| A | JP 2008-533491 A (BIOTIUM, INC.) 21 August 2008 (2008-08-21) claims, examples | 1-9 |
| A | US 2021/0198729 A1 (BIOACTS CORPORATION) 01 July 2021 (2021-07-01) claims, examples | 1-9 |
| A | US 2016/0024067 A1 (BIOTIUM, INC.) 28 January 2016 (2016-01-28) claims, examples | 1-9 |
| A | US 2013/0137875 A1 (YING, Laiqiang) 30 May 2013 (2013-05-30) claims, examples | 1-9 |
| A | CN 111349091 A (BEIJING FLUORESCENCE BIOTECHNOLOGY CO., LTD.) 30 June 2020 (2020-06-30) claims, examples | 1-9 |

☐ Further documents are listed in the continuation of Box C.    ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **18 April 2024** | **14 May 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2024/006954**

| Patent document cited in search report | Publication date (day/month/year) | Patent family member(s) | Publication date (day/month/year) |
|---|---|---|---|
| JP 2008-533491 A | 21 August 2008 | US 2006/0211028 A1<br>claims, examples<br>US 2006/0211029 A1<br>US 2010/0009454 A1<br>US 2010/0317016 A1<br>US 2010/0323453 A1<br>US 2013/0167309 A1<br>US 9102835 B2<br>US 2014/0106349 A1<br>WO 2006/099605 A2<br>EP 2428586 A1<br>CA 2601455 A1<br>CN 101142326 A<br>CN 102942566 A<br>KR 10-2007-0116118 A | |
| US 2021/0198729 A1 | 01 July 2021 | KR 10-2230263 B1 | |
| US 2016/0024067 A1 | 28 January 2016 | WO 2014/005125 A2 | |
| US 2013/0137875 A1 | 30 May 2013 | (Family: none) | |
| CN 111349091 A | 30 June 2020 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5658751 A


### Non-patent literature cited in the description


- *J. Fluoresc.*, 2012, vol. 22, 1189-1199
- *Nucleic Acid Research*, 2021, vol. 49, 5143-5158
- *Sci Transl Med*, 07 November 2018, vol. 10 (466)